# EUROPEAN PATENT SPECIFICATION

(11) **EP 2 163 251 B1**
(45) Date of publication and mention of the grant of the patent: **29.08.2012**
(21) Application number: 08016054.2
(22) Date of filing: 11.09.2008
(51) Int. Cl.: A61K 35/26, A61P 43/00

(54) **Treatment of ascites with thymus extracts**
Behandlung von Aszites mit Thymusdrüsenextrakten
Traitement d'ascite avec des extraits de thymus

(43) Date of publication of application: 17.03.2010
(73) Proprietor: Löhr, Katrin, 50672 Köln (DE)
(72) Inventor: Dr. Günther Löhr, 79241 Ihringen (DE)
(74) Representative: Maucher, Wolfgang

(56) References cited:
- CHUNG MICHAEL ET AL: "Treatment of malignant ascites." CURRENT TREATMENT OPTIONS IN ONCOLOGY JUN 2008, vol. 9, no. 2-3, June 2008 (2008-06), pages 215-233, XP002517783 ISSN: 1534-6277
- ZERVOS E E ET AL: "Management of medically refractory ascites." AMERICAN JOURNAL OF SURGERY MAR 2001, vol. 181, no. 3, March 2001 (2001-03), pages 256-264, XP002517784 ISSN: 0002-9610
- SPANGENBERG H C ET AL: "[Systemic treatment for hepatocellular carcinoma]" ZENTRALBLATT FÜR CHIRURGIE NOV 2003, vol. 128, no. 11, November 2003 (2003-11), pages 906-910, XP009113028 ISSN: 0044-409X
- DOLLINGER MATTHIAS M ET AL: "Thymostimulin in advanced hepatocellular carcinoma: A phase II trial" BMC CANCER, BIOMED CENTRAL, LONDON, GB, vol. 8, no. 1, 13 March 2008 (2008-03-13), page 72, XP021034707 ISSN: 1471-2407
- CHIEN RONG-NAN ET AL: "Thymalfasin for the treatment of chronic hepatitis B." EXPERT REVIEW OF ANTI-INFECTIVE THERAPY FEB 2004, vol. 2, no. 1, February 2004 (2004-02), pages 9-16, XP002517785 ISSN: 1478-7210

## Description

The present invention relates to a novel use of thymus extracts, corresponding methods, and the extracts for use in said novel use.

Extracts from thymus, such as thymostimulin (sold under the trademark THYMOPHYSIN® by CytoChemia AG, Ihringen, Germany) or TP-1 (Serono, see e.g. DE 34 30 375), are known to be useful as e.g. parenteral nutritional supplements, e.g. in case of cachexia, malnutrition and tumor anorexia, as well as for use as immunostimulants, e.g. in the treatment of weakness of the immune system, for example as consequence of infectious diseases, such as HIV infection (e.g. in AIDS treatment).

Hepatocellular carcinoma (HCC) is a disease that currently ranks fifth among the most common malignancies world-wide with a rising incidence in first-world countries1. Although treatment options have become more diverse in recent years, improvements in survival rates lack far behind those achieved in other tumor entities. Benefiting most of newer modalities are patients amenable to local therapy, i.e. with intermediate stages of the disease, small tumors and preserved liver function. In contrast, patients with advanced and metastatic HCC or recurrent tumors after first-line therapy remain without proven standard treatment resulting in a life expectancy of less than 10% at 3 years. Most systemic approaches have yielded disappointing results despite major side-effects.

In an attempt to find further useful means against this disease, immunostimulation has been considered as a possible option to be scrutinized. In Phase II clinical trials, some beneficial effect could be found using thymostimulin as active principle (see Dollinger M.M. et al., BMC Cancer, 8:72, 2008, doi:10.1186/1471-2407-8-72, published on March 13, 2008).

Ascites is known as peritoneal cavity fluid, peritoneal fluid excess, hydroperitoneum or formerly as abdominal dropsy. It is characterized by an accumulation of fluid in the peritoneal cavity. Although most commonly due to cirrhosis and severe liver disease, its presence can also be due to some other significant medical disorders. Treatment so far is made with medication (diuretics), by paracentesis or by other treatments directed at the cause.

It would be useful to find active principles that allow for the treatment of ascites caused by many of the mentioned or other diseases or disorders or without known ethilogy.

It has now surprisingly been found that in a Phase III clinical study on the treatment of HCC a beneficial effect of thymus extracts on ascites, a symptome or condition also accompanying HCC, could be found, irrespective of whether or not an effect on HCC itself could be found.

The invention thus relates, in a first embodiment, to a mammalian thymus extract for use in the treatment of ascites, irrespective of the cause for said ascites, preferably if found in parallel to HCC.

In a second embodiment, the invention relates to the extract for use mentioned in the last paragraph wherein the thymus extract also has immunopotentiating activity.

In a third embodiment, the invention relates to the extract for use according to the preceding two paragraphs, wherein the thymus extract is an extract from bovine thymus.

In fourth embodiment, the invention relates to the extract for use according to any one of the preceding three paragraphs where the thymus extract is a peptide extract from bovine thymus.

In a fifth embodiment, the invention relates to the extract for use according to any one of the preceding four paragraphs, wherein the thymus extract is characterised by consisting substantially of molecules with a molecular weight between 500 and 20 000 Dalton, more preferably between 1000 and 10 000 Dalton.

"Substantially" means that at least 60, preferably at least 70, more preferably at least 80, more preferably at least 90, more preferably at least 95 % of the molecules fulfil the definition.

In a sixth embodiment, the invention relates to the extract for use according to any one of the preceding five paragraphs in the treatment of ascites as accompanying symptom of hepatocellular carcinoma.

In a seventh embodiment, the invention relates to the extract for use according to any one of the preceding six paragraphs, where the extract is characterized by two main characteristic bands on a polyacrylamide gel at pH 8.6 with an R_{f} of about 0.25 and 0.44, respectively. This is especially the case for TP-1 described in DE 34 30 375.

In an eighth embodiment, the invention thus relates to the extract for use according to any one of the preceding seven paragraphs, where the extract is TP-1.

In a ninth embodiment, the invention relates to the extract for use according to any one of the seven paragraphs preceding the immediately preceding paragraph, where the extract is a low molecular weight thymus fraction from calf thymus, preferably thymostimulin.

In a tenth embodiment, the invention relates to the extract for use according to any one of the preceding nine paragraphs, where the extract is in the form of a pharmaceutical preparation for parenteral use.

In an eleventh embodiment, the invention relates to a pharmaceutical formulation for use according to any one of the preceding ten paragraphs, **characterized in that** it comprises an extract for use according to any one of the ten preceding paragraphs and a pharmaceutically acceptable carrier material.

In a twelfth embodiment, the invention relates to the use of an extract from mammalian thymus for the preparation of a pharmaceutical preparation useful in the treatment of ascites.

In a thirteenth embodiment, the invention relates to the use according to the preceding paragraph, where the extract is an extract according to any one of the second to tenth embodiment mentioned above.

Thymus extract are preferred which comprise a protein or especially one or more lower molecular weight peptide fractions obtained from mammalian thymus, especially bovine thymus, most especially calf thymus.

Corresponding methods for their isolation are known in the art, for example as described in DE 34 30 375 and the references quoted therein.

In a preferred embodiment, the thymus extract is prepared as follows:
Thymostimulin is an immunomodulating drug prepared from an extract of peptides from bovine thymus glands (Thymophysin CytoChemia® 25/50). Following removal of higher molecular cell components and proteins, low-molecular active thymus peptides are isolated and standardized to a defined protein fraction.

In brief detail, the extract can be prepared as follows:
In a first step, homogenized calf thymus is extracted with an ammonium acetate solution. The resulting supernatant is precipitated twice with ammonium sulphate. After that, dialysis follows for desalting, removing molecules with less than 1000 Dalton molecular weight. This is followed by ultrafiltration of the resulting solution to give a solution of components having 10 000 Dalton or less. This resulting solution is then lyophilized to give the final active principle.

Administration of a thymus extract according to the invention preferably takes place enterally, e.g. orally, rectally or nasally, but more preferably parenterally, e.g. by infusion or advantageously simply by injection, e.g. s.c., i.m. or i.p. injection.

The general terms used hereinbefore and hereinafter preferably have within the context of this disclosure the following meanings, unless otherwise indicated, where more general terms whereever used may, independently of each other, be replaced by more specific definitions or remain, thus defining more preferred embodiments of the invention:
The subject to be treated according to the embodiments of the invention is preferably a human, most preferably a human in need of such treatment, e.g. suffering from ascites.

The term "treatment", wherever mentioned, comprises the curative or palliative treatment, as well as prophylactic treatment in patients where ascites is to be expected. Especially it means a treatment at least temporarily mitigating ascites or preventing or delaying its onset.

"Useful in the treatment" means especially prophylactically or therapeutically effective, especially as defined in the preceding paragraph.

The dosage of the active ingredient (thymus extract) depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The dose of the active ingredient to be administered to warm-blooded animals, for example humans of approximately 70 kg body weight, is preferably from approximately 10 mg to approximately 250 mg, more preferably from approximately 20 mg to approximately 150 mg per person per day, in one dose or divided into more doses, e.g. up to three doses per day. Preferably, the treatment is repeated daily, but may also be repeated on 3 to 6, such as 5, days per week. Preferred is dosing in the form of parenteral compositions.

The pharmaceutical formulation according to the invention may comprise the active principle in combination with one or more pharmaceutically acceptable carrier materials (= excipients).

Pharmaceutical compositions according to the invention may be, for example, in unit dose form, such as in the form of ampoules, vials, or further dragées, tablets or capsules.

Preferably, the formulation is a parenteral formulation in dry form (then to be mixed with an appropriate solution, such as physiological saline solution, before use) or in liquid form (solution). Most preferred is an injection solution.

Solutions of the active ingredient, and also suspensions, and especially isotonic aqueous solutions or suspensions, are useful for parenteral administration of the active ingredient, it being possible, for example in the case of lyophilized compositions that comprise the active ingredient alone or together with a pharmaceutically acceptable carrier, for example sodium chloride or mannitol, for such solutions or suspensions to be produced prior to use. The pharmaceutical compositions may be sterilized and/or may comprise excipients, for example preservatives, stabilizers, wetting and/or emulsifying agents, solubilizers, salts for regulating the osmotic pressure and/or buffers, and are prepared in a manner known per se, for example by means of conventional dissolving or lyophilizing processes. The solutions or suspensions may comprise viscosity increasing substances, such as sodium carboxymethylcellulose, carboxymethylcellulose, dextran, polyvinylpyrrolidone or gelatin.

Suspensions in oil comprise as the oil component the vegetable, synthetic or semi-synthetic oils customary for injection purposes.

The injection or infusion compositions (dry form or solution) are prepared in customary manner under sterile conditions; the same applies also to introducing the compositions into ampoules or vials and sealing the containers.

An injection or infusion solution preferably must preferably have the same or essentially the same osmotic pressure as body fluid. Accordingly, the aqueous medium preferably contains an isotonic agent which has the effect of rendering the osmotic pressure of the infusion solution the same or essentially the same as body fluid.

The isotonic agent may be selected from any of those known in the art, e.g. mannitol, dextrose, glucose and sodium chloride. Preferably the isotonic agent is glucose or sodium chloride. The isotonic agents may be used in amounts which impart to the infusion solution the same or essentially the same osmotic pressure as body fluid. The precise quantities needed can be determined by routine experimentation and will depend upon the composition of the injection or infusion solution and the nature of the isotonic agent. Selection of a particular isotonic agent can be made having regard to the properties of the active agent.

The concentration of isotonic agent in the aqueous medium will depend upon the nature of the particular isotonic agent used. When the isotonic agent is sodium chloride it is preferably employed in amounts of up to 1% w/v, in particular up to 0.9% w/v.

The formulation may be diluted with the aqueous medium. The amount of aqueous medium employed as a diluent is chosen according to the desired concentration of active ingredient in the injection or infusion solution. Preferably the infusion or especially injection solution is made by mixing a vial or ampoule of dry concentrate with an aqueous medium, making the volume up to between 1 ml and 200 ml, preferably between about 1ml and about 5 ml, with the aqueous medium.

Injection or infusion solutions may contain other excipients commonly employed in formulations to be administered intravenously or by injection. Excipients include antioxidants. Injection or infusion solutions may be prepared by mixing an ampoule or vial of the formulation with the aqueous medium, e.g. a 0.9% sodium chloride solution in a suitable container, e.g. an infusion bag or bottle or preferably an ampoule. The injection or infusion solution, once formed, is preferably used immediately or within a short time of being formed, e.g. within 6 hours. Containers for holding the injection or infusion solutions may be chosen from any conventional container which is nonreactive with the injection or infusion solution. Glass containers are suitable although it may be preferred to use plastics containers, e.g. plastics infusion bags or ampoules.

Pharmaceutical compositions for enteral, e.g. oral or nasal or inhalatory administration, can be obtained by combining the active ingredient with solid carriers, if desired granulating a resulting mixture, and processing the mixture, if desired or necessary, after the addition of appropriate excipients, into tablets, dragée cores or capsules, or be filled into a powder inhalator for administration by inhalation. It is also possible for them to be incorporated into plastics carriers that allow the active ingredients to diffuse or be released in measured amounts.

The following Example, while showing the efficiency of thymus extracts in an illustrative way, is not limiting the scope of the present invention.

### Example: Phase III Study

A study was designed as a prospective, randomised, controlled, double-blind, multi-centre phase III trial comparing treatment of HCC with thymostimulin to placebo. Patients were recruited at 12 centres in Germany (see Acknowledgements). Primary endpoint of the study was 6- and 12-month survival, secondary endpoints overall survival, tumor response and progression-free survival according to standard WHO criteria¹, toxicity according to ECOG criteria² and quality of life assessed by means of the FACT-Hep questionnaire³. A sample size of 110 patients was calculated necessary to detect a 20% improvement in one-year-survival in a two-sided test with a significance level of 5% and a power of 90%. Allowing for a dropout rate of at least 20%, 135 patients were planned to be recruited within 24 months. After inclusion of the last patient, a follow-up period of 12 months was scheduled.

Using external randomization covariate-adapted to each centre and performed centrally via fax, patients were assigned to thymostimulin 75 mg subcutaneously for 5 days a week according to manufacturer's specifications or placebo for one year in addition to best supportive care as required. Study medication (verum and placebo) was coded and labeled to guarantee blinding with the first five injections performed by a medical doctor. Further injections were administrated by the patients themselves or home care workers and registered in a treatment diary. Drug accountability procedures, reporting of (serious) adverse events and source data verification were implemented as part of the trial monitoring according to standard operating procedures conducted by external monitors. Treatment with the study medication was continued for one year or until one of the following criteria was met: disease progression, decompensation of liver function (bilirubin > 5 mg/dl, INR ≥ 2.3) or performance status (Karnofsky score < 50%), unacceptable toxicity, patient refusal or incompliance.

Pre-treatment and follow-up evaluation included a complete medical history, physical examination, blood count and chemistry as well as performance status. Risk factors and extent of liver disease according to Child-Pugh status as well as prior treatment modalities were recorded at baseline. Tumors were assessed by abdominal ultrasound, chest X-ray and either dynamic computerized tomography (CT) or magnetic resonance imaging (MRI) with other imaging such as radionuclide scans employed if clinically indicated. Okuda-, CLIP- and BCLC-classifications were used for staging. Follow-up investigations without imaging were conducted every 6 weeks including survival data as well as documentation of concomitant therapies and toxicity of the medication. Tumor response using CT or MRI scanning evaluated according to WHO criteria' and quality of life assessed by means of the FACTHep questionnaire³ were measured every 3 months. This type of follow-up was scheduled for at least one year after enrollment, followed by three-monthly assessments in surviving patients.

Statistical analysis were by intention to treat. Comparisons of continuous variables were done by the Wilcoxon rank-sum method and for categorical variables by the Fisher's exact test, in case of adverse events by the Chi-square test. Survival time and progression-free survival were calculated from the start of therapy to the date of death or date of progression/death without progression, respectively. Survival curves were established with the Kaplan-Meier method. A stepwise forward Cox's regression analysis of survival was used to assess baseline predictors and the treatment effect simultaneously. The following variables were chosen for the univariate analysis: age, gender and Karnofsky score; the presence of liver cirrhosis and Child classification; Okuda-, CLIP- and BCLC-classification; AFP-level, multifocal tumor manifestation, ascites, vascular invasion and extrahepatic metastases; treatment with thymostimulin or placebo and treatment with other therapy modalities before study entry. Significant variables in the univariate analysis were introduced into the multivariate analysis. Calculations were done with the SPSS package (version 15.0).

### Results:

### Patients

A total of 135 patients were enrolled between October 2002 and March 2005, and randomly assigned to treatment with thymostimulin (n=67) or placebo (n=68). Detailed demographic data and tumor-related characteristics are depicted in Table 1. Most patients had liver cirrhosis and the majority of tumors was staged as advanced HCC (Okuda stage II; CLIP 2-3 points; BCLC stage C) with multinodular tumor growth, vascular invasion and extrahepatic spread. Less than a third of patients had been treated prior to enrollment with surgical resection (R1 or R2 resection), PEI (range 1-5 sessions), TACE (range 1-7 sessions) or systemic hormone- and/or chemotherapy (somatostatin in 7/14 cases; single or combination therapy with tamoxifen, doxorubicin or platin derivates), but suffered from tumor progression. No significant imparity in baseline characteristics was detected between the two groups indicating a successful randomization process. Following randomization, 6 patients in the thymostimulin group and 1 patient in the placebo group did not receive the allocated treatment. Their data was censored at the time of discontinuation of follow-up and included in the intention-to-treat analysis. A subgroup analysis excluding these patients did not reveal a different outcome (data not shown). The remaining patients received treatment as scheduled, in most cases until disease progression or deterioration of liver function or performance status. One patient in each group received salvage therapy after tumor progression, in the placebo group TACE, in the thymostimulin group tamoxifen. Secondary tumors requiring alternative therapy were also diagnosed one in each group; in the thymostimulin group, treatment of the patient had to be stopped early due to a hypopharyngeal cancer, in the placebo group the secondary urothelial cancer was detected after 12 months of treatment of the patient. Only 12 patients (thymostimulin, n=7 [10%]; placebo, n=5 [7%]) received the study medication for a whole year. At the time of analysis, all patients had stopped their allocated treatment. The median follow-up was 5.2 months (95% CI 4.0-7.3) in the thymostimulin group and 5.6 months (95% CI 3.6-7.3) in the placebo group, the median length of treatment 3.3 months (range 0 to 12 months) for both groups.

### Survival and tumor response

For the thymostimulin group, the probability of survival at 6 and 12 months was 40% and 22%, respectively, with a median survival time of 5.0 months (95% CI 3.7-6.3). There was no difference in survival compared with the placebo group with a 6- and 12-month survival of 38% and 21%, respectively, and a median survival time of 5.2 months (95% CI 3.5-6.9). Baseline predictors and their effect on survival in the two groups were assessed using a uni- and multivariate analysis. Thus, adjustment for age, gender, stage of liver disease (Child classification), tumor stage (Okuda-, CLIP- and BCLC-classification), invasive tumor phenotype (vascular invasion or metastases) or treatment prior to enrollment did not affect outcome and the lack of a difference in survival between groups. In contrast, all three classifications of tumor stage proved their prognostic power for HCC in the univariate analysis, although in the multivariate analysis the CLIP-classification alone remained independently related to survival in our population (Table 2). The analysis of the objective tumor response (complete response: disappearance of all demonstrable disease; partial response: decrease ≥ 50% of the longest diameter of the target lesion without worsening of all other disease; stable disease: no significant change in disease [decrease < 50% or increase < 25% without new lesions]; progressive disease: increase ≥ 25% of the longest diameter of the target lesion or new lesions) showed no complete response in any of the patients. 22 (33%) out of 67 patients in the thymostimulin group achieved stable disease or a partial response in the first 6 months followed by progressive disease thereafter. However, there was a similar tumor response rate in the placebo group in 20 (29%) out of 68 patients, and the progression-free survival did not differ between groups with a median of 5.3 months (95% CI 2.0-8.6; thymostimulin) and 2.9 months (95% CI 2.6-3.1; placebo), respectively.

### Safety

Compliance with the allocated treatment was high with an average of 94% and 92% of patients receiving all 5 injections a week in every 6 week-cycle in the thymostimulin and placebo group, respectively (n.s.). While skin reactions following injection are the only known side-effects of thymostimulin, it has been shown to reduce chemotherapy-induced toxicity, in particular myelosuppression and infectious episodes⁴. In our study, 219 adverse events were reported - 95 in the placebo and 124 in the thymostimulin group (n.s.). Although not significantly more common in either group, skin reactions caused the early withdrawal from the trial of two patients on thymostimulin and one patient on placebo. Only one more patient on placebo withdrew due to adverse events, i.e. reproducible diarrhea after injection. 139/219 adverse events were classified as serious - 85 in the placebo and only 54 in the thymostimulin group.

The difference regarding the serious adverse events was caused by a significantly lower incidence of hepato-renal deterioration with ascites and renal failure typically associated with end-stage liver disease in patients treated with thymostimulin (Table 3). In contrast, hematological or infectious complications were equally common in both groups.

In summary, the treatment of HCC in the present design of the Phase III study was not significantly successful. As single agent, thymostimulin has no proven cytotoxic effect on patients with large, advanced tumors, who are most at need of new therapeutical approaches.

However, a significant observation was made that thymostimulin allowed to positively affect ascites in patients suffering from HCC.

In contrast to the lacking influence on tumor growth, thus, a beneficial effect of thymostimulin on hepato-renal function of our study patients was found. With their excellent safety record, only skin reactions following injection can be related as side-effects to thymosin-α1 containing compoundss^{5,6}. In addition, several studies have explored their potential in preventing side-effects of other chemotherapeutic agents, in particular myelosuppression and infectious complications^{4,7,8}. Protection against renal failure, though, has only been observed to date in an animal model of experimentally induced uremia using thymosin fraction 5 (TF5)⁹.

In patients with advanced HCC, deterioration of hepato-renal function with growing ascites and renal failure is a common complication of the end-stage liver disease. It is therefore tempting to speculate that any improvement of survival previously seen in HCC patients using thymostimulin or thymosin-α1 containing compounds is in part caused by preventing hepato-renal decompensation.

### Tables:

**Table 1: Baseline characteristics of patients**

| Baseline characteristics | Placebo n=68 | Thymostimulin n=67 |
|---|---|---|
| Patients | | |
| Male/female (%) | 82/18 | 84/16 |
| Median age | | |
| (years (range)) | 63 (39-76) | 63 (48-79) |
| Median body weight (kg (range)) | 80 (39-108) | 79 (58-112) |
| Mean Karnofsky score (% (range)) | 87 (60-100) | 89 (60-100) |

| Cause of liver disease (%) | | |
|---|---|---|
| Alcohol abuse | 47 | 55 |
| HBV/HCV | 21 | 18 |
| Other/unknown | 32 | 27 |

| Stage of liver disease (%) | | |
|---|---|---|
| Liver cirrhosis | 90 | 85 |
| Child classification: no cirrhosis or A/B/C | 63/28/9 | 65/32/3 |

| Tumor stage (%) | | |
|---|---|---|
| Okuda stage I/II/III | 32/56/12 | 35/54/11 |
| CLIP score 0/1/2/3/4- | 0/18/35/25/22 | 0/15/29/29/27 |

| 6 BCLC stage A/B/C/D | 0/16/69/15 | 0/22/66/12 |
|---|---|---|
| α-FP (%) | | |
| < 400 ng/ml | 57 | 57 |
| > 400 ng/ml | 43 | 43 |

| Tumor characteristics (%) | | |
|---|---|---|
| Ascites | 46 | 37 |
| Portal vein thrombosis | 32 | 42 |
| Multifocal tumor manifestation | 88 | 91 |
| Extrahepatic metastases | 40 | 45 |

| Hepato-renal laboratory parameters | | |
|---|---|---|
| Mean urea | | |
| (mg/dl [95% CI]) | 4.3 [2.8-5.8] | 3.7 [2.8-4.5] |
| Mean creatinine (mg/dl [95% CI]) | 0.4 [0.3-0.6] | 0.5 [0.4-0.6] |
| Mean sodium | | |
| (mmol/l [95% CI]) | 137 [136-138] | 137 [136-138] |
| Mean albumin | | |
| (g/l [95% CI]) | 27 [23-31] | 29 [26-32] |

| Previous treatment (%) | | |
|---|---|---|
| (combination possible) | 68 15 | 72 9 |
| none Surgery | 4 | 2 |
| Percutaneous ethanol injection (PEI) | | |
| Transarterial chemoembolisation | 25 | 20 |
| (TACE) | 12 | 9 |
| Systemic hormone- and/or chemotherapy | | |

| | | |
|---|---|---|
| Note: There were no significant differences between the groups | | |

**Table 2: 6- and 12-month survival of all patients (n = 135) according to their tumor stage and prognostic relevance of each classification**

| **Tumor classification** | **No. of patients (%)** | **6-/12-month survival (%)** | | | **univariate analysis** | **multivariate analysis** |
|---|---|---|---|---|---|---|
| **Okuda stage I** | 34 | | 56 | | p < 0.0001 | p = 0,266 |
| **Okuda stage II** | 55 | | 31 | | | |
| **Okuda stage III** | 11 | | 37 | | | |
| | | | 21 | | | |
| | | 7 | | 0 | | |
| CLIP 0 | / | / | | / | p < 0.0001 | p = 0.013 |
| CLIP 1 | 17 | 68 | | 46 | | |
| CLIP 2 | 32 | 51 | | 35 | | |
| CLIP 3 | 27 | 33 | | 11 | | |
| CLIP 4-6 | 24 | 13 | | 0 | | |
| BCLC stage A | / | / | | / | p < 0.0001 | p = 0.133 |
| BCLC stage B | 19 | 68 | | 48 | | |
| BCLC stage C | 68 | 37 | | 18 | | |
| BCLC stage D | 13 | 17 | | 6 | | |

**Table 3 (excerpt): Comparison between groups of (serious) adverse events (AE) graded according to ECOG criteria and occurring in more than 2% of all patients (n=135; significance level p < 0.05).**

| **ECOG toxicity criteria** | **Placebo (n=68)** | | | | **Thymostimulin (n=67)** | | | | **p-value** | |
|---|---|---|---|---|---|---|---|---|---|---|
| | Grade 1 | Grade 2 | Grade 3 | Grde 4 | Grade 1 | Grade 2 | Grade 3 | Grade 4 | all AE | Grade ¾ AE |
| **Gastrointestinal/ Hepatic** | | | | | | | | | | |
| Diarrhoea | 1 | | 2 | | 2 | 2 | | | 0,6831 | 0,1573 |
| Hyperbilirubinaemia | | | | | | | | | 0,5681 | 0,5681 |
| Elevated | | | | 2 | | | | 1 | | |
| transaminases | | 1 | 2 | 1 | 2 | 1 | 2 | | 0,7128 | 0,6608 |
| GI haemorrhage | | | 4 | 2 | | | 4 | 6 | 0,2728 | 0,2728 |
| Ascites (without renal failure) | | 1 | 14 | 3 | | 2 | 6 | | **0,0323** | **0,0132** |
| | | | | | | | | | | |
| Renal Elevated creatinine or urea | 5 | 2 | | | 1 | | | 1 | 0,0887 | 0,3119 |
| Proteinuria | 2 | | | | | | | | 0,1573 | - |
| Renal failure (with ascites) | | | 7 | 5 | | | | 2 | **0,0052** | **0,0052** |
| | | | | | | | | | | |

Literature quoted:
1) Miller AB, Hoogstraten B, Staquet M, Winkler A. Reporting results of cancer treatment. Cancer 1981; 47:207-214
2) Oken MM, Creech RH, Tormey DC, Horton J, Davis TE, McFadden ET, Carbone PP. Toxicity and response criteria of the Eastern Cooperative Oncology Group. Am J Clin Oncol 1982; 5:649-655.
3) Heffernan N, Cella D, Webster K, Odom L, Marone M, Passik S, Bookbinder M, Fong Y, Jarnagin W, Blumgart L. Measuring health-related quality of life in patients with hepatobiliary cancers: The Functional Assessment of Cancer Therapy - Hepatobiliary (FACT-Hep) questionnaire. J Clin Oncol 2002; 20:2229-2239.
4) Macchiarini P, Danesi R, Del Tacca M, Angeletti CA. Effects of thymostimulin on chemotherapy-induced toxicity and long-term survival in small cell lung cancer patients. Anticancer Res 1989; 9:193-6.
5) Rustigi VK. Thymalfasin for the Treatment of Chronic Hepatitis C Infection. Ann NY Acad Sci 2007; 1112:357-367.
6) Garaci E, Favalli C, Pica F, Vallebona PS, Palamara AT, Matteucci C, Pierimarchi P, Serafino A, Mastino A, Bistoni F, Romani L, Rasi G. Thymosin Alpha 1: From Bench to Bedside. Ann NY Acad Sci 2007; 1112:225-234.
7) Tortorella C, Ottolenghi A, Moretti AM, Jirillo E, Antonaci S. Thymostimulin administration modulates polymorph metabolic pathway in patients with chronic obstructive pulmonary disease. Immunopharmacol Immunotoxicol 1992; 14:421-437.
8) Romani L, Bistoni F, Montagnoli C, Gaziano R, Bozza S, Bonifazi P, Zelante T, Moretti S, Rasi G, Garaci E, Puccetti P. Thymosin α1: An Endogenous Regulator of Inflammation, Immunity, and Tolerance. Ann NY Acad Sci 2007; 1112:326-338.
9) Ikemoto S, Sugimura K, Kamizuru M, Wada S, Yasumoto R, Yamamoto K, Kishimoto T. Protective effect of thymosin fraction 5 in animals with experimentally induced uremia.Clin Exp Nephrol 2000; 4:215-219.

## Claims

1. A mammalian thymus extract for use in the treatment of ascites.

2. The extract for use according to claim 1, wherein the thymus extract has immunopotentiating activity.

3. The extract for use according to claim 1 or claim 2, wherein the thymus extract is an extract from bovine thymus.

4. The extract for use according to any one of claims 1 to 3 where the thymus extract is a peptide extract from bovine thymus.

5. The extract for use according to any one of claims 1 to 4, wherein the thymus extract is **characterised by** a molecular weight substantially between 1000 and 10 000 Dalton.

6. The extract for use according to any one of claims 1 to 5 in the treatment of ascites as accompanying symptom of hepatocellular carcinoma.

7. The extract for use according to any one of claims 1 to 6, where the extract is **characterized by** two main characteristic bands on a polyacrylamide gel at pH 8.6 with an R_{f} of about 0.25 and 0.44, respectively.

8. The extract for use according to any one of claims 1 to 7, where the extract is TP-1.

9. The extract for use according to any one of claims 1 to 7, where the extract is a low molecular weight thymus fraction from calf thymus, preferably thymostimulin.

10. The extract for use according to any one of claims 1 to 9, where the extract is in the form of a pharmaceutical preparation for parenteral use.

11. A pharmaceutical formulation for use according to any one of claims 1 to 10, **characterized in that** it comprises an extract for use according to any one of claims 1 to 10 and a pharmaceutically acceptable carrier material.

12. The use of an extract from mammalian thymus for the preparation of a pharmaceutical preparation for the treatment of ascites.

13. The use according to claim 12, where the extract is an extract according to any one of claims 2 to 10.

## Patentansprüche

1. Säugetierthymusextrakt zur Verwendung bei der Behandlung von Aszites.

2. Extrakt zur Verwendung nach Anspruch 1, wobei der Thymusextrakt eine immunpotenzierende Wirkung hat.

3. Extrakt zur Verwendung nach Anspruch 1 oder 2, wobei es sich bei dem Thymusextrakt um einen Extrakt aus Rinderthymus handelt.

4. Extrakt zur Verwendung nach einem der Ansprüche 1 bis 3, wobei es sich bei dem Thymusextrakt um einen Peptidextrakt aus Rinderthymus handelt.

5. Extrakt zur Verwendung nach einem der Ansprüche 1 bis 4, wobei der Thymusextrakt durch ein Molekulargewicht von im Wesentlichen zwischen 1000 und 10 000 Dalton charakterisiert ist.

6. Extrakt zur Verwendung nach einem der Ansprüche 1 bis 5 bei der Behandlung von Aszites als Begleitsymptom von hepatozellulärem Karzinom.

7. Extrakt zur Verwendung nach einem der Ansprüche 1 bis 6, wobei der Extrakt durch zwei charakteristische Hauptbanden auf einem Polyacrylamidgel bei einem pH-Wert von 8,6 mit einem R_{f} von etwa 0,25 bzw. 0,44 gekennzeichnet ist.

8. Extrakt zur Verwendung nach einem der Ansprüche 1 bis 7, wobei es sich bei dem Extrakt um TP-1 handelt.

9. Extrakt zur Verwendung nach einem der Ansprüche 1 bis 7, wobei es sich bei dem Extrakt um eine niedermolekulare Thymusfraktion aus Kälberthymus, vorzugsweise Thymostimulin, handelt.

10. Extrakt zur Verwendung nach einem der Ansprüche 1 bis 9, wobei der Extrakt in Form einer pharmazeutischen Zubereitung für die parenterale Anwendung vorliegt.

11. Pharmazeutische Formulierung zur Verwendung nach einem der Ansprüche 1 bis 10, **dadurch gekennzeichnet, dass** sie einen Extrakt zur Verwendung nach einem der Ansprüche 1 bis 10 und ein pharmazeutisch unbedenkliches Trägermaterial umfasst.

12. Verwendung eines Extrakts aus Säugetierthymus zur Herstellung einer pharmazeutischen Zubereitung zur Behandlung von Aszites.

13. Verwendung nach Anspruch 12, wobei es sich bei dem Extrakt um einen Extrakt nach einem der Ansprüche 2 bis 10 handelt.

## Revendications

1. Extrait de thymus de mammifère pour emploi dans le traitement de l'ascite.

2. Extrait pour emploi conforme à la revendication 1, où l'extrait de thymus présente une activité d'immunopotentiation.

3. Extrait pour emploi conforme à la revendication 1 ou à la revendication 2, où l'extrait de thymus est un extrait de thymus bovin.

4. Extrait pour emploi conforme à l'une quelconque des revendications 1 à 3, où l'extrait de thymus est un extrait peptidique de thymus bovin.

5. Extrait pour emploi conforme à l'une quelconque des revendications 1 à 4, où l'extrait de thymus est **caractérisé par** une masse moléculaire substantiellement comprise entre 1000 et 10 000 daltons.

6. Extrait pour emploi conforme à l'une quelconque des revendications 1 à 5 dans le traitement de l'ascite en tant que symptôme accompagnant un carcinome hépatocellulaire.

7. Extrait pour emploi conforme à l'une quelconque des revendications 1 à 6, où l'extrait est **caractérisé par** deux bandes principales caractéristiques sur gel de polyacrylamide à pH 8,6 à des R_{f} respectifs d'environ 0,25 et 0,44.

8. Extrait pour emploi conforme à l'une quelconque des revendications 1 à 7, où l'extrait est TP-1.

9. Extrait pour emploi conforme à l'une quelconque des revendications 1 à 7, où l'extrait est une fraction de thymus de faible masse moléculaire issue de thymus de veau, préférentiellement la thymostimuline.

10. Extrait pour emploi conforme à l'une quelconque des revendications 1 à 9, où l'extrait est sous forme d'une préparation pharmaceutique à usage parentéral.

11. Formule pharmaceutique pour emploi conforme à l'une quelconque des revendications 1 à 10, **caractérisée en ce qu'**elle comprend un extrait pour emploi conforme à l'une quelconque des revendications 1 à 10 et une substance vectrice de qualité pharmaceutique.

12. Emploi d'un extrait de thymus de mammifère dans l'élaboration d'une préparation pharmaceutique destinée au traitement de l'ascite.

13. Emploi conforme à la revendication 12, où l'extrait est un extrait conforme à l'une quelconque des revendications 2 à 10.
